# EUROPEAN PATENT APPLICATION

(11) **EP 4 159 152 A1**
(43) Date of publication of application: **05.04.2023**
(21) Application number: 21812475.8
(22) Date of filing: 28.05.2021
(51) Int. Cl.: A61B 34/30, A61B 90/00

(54) **POSITIONING DEVICE AND POSITIONING METHOD FOR MECHANICAL ARM OF SURGICAL ROBOT**

(30) Priority: 29.05.2020 CN 202010474025
(71) Applicant: Suzhou Kangduo Robot Co., Ltd., Suzhou, Jiangsu 215153 (CN)
(72) Inventor: WANG, Jianguo, Suzhou, Jiangsu 215153 (CN); WANG, Xiaowei, Suzhou, Jiangsu 215153 (CN)
(74) Representative: Zaboliene, Reda
(86) International application number: PCT/CN2021/096983
(87) International publication number: WO 2021/239139

(57) **Abstract**

The invention provides a positioning device and a positioning method for a mechanical arm of a surgical robot, and belongs to the technical field of medical instruments. The positioning device for a mechanical arm of a surgical robot includes a poking card holder for being connected to an end of a mechanical arm, and an auxiliary positioning unit detachably connected to the poking card holder; and the auxiliary positioning unit is used to simulate a part of a surgical poking card located above a surgical poking card identification line to determine an accurate position where the mechanical arm is connected with the surgical poking card. The positioning device and the positioning method for a mechanical arm of a surgical robot in the invention can avoid the risk of secondary trauma to a patient's abdominal wall wound during the adjustment of the mechanical arm, and realize the quick connection between the mechanical arm and the surgical poking card, which greatly shortens the preparation time before surgery.

## Description

### Technical Field

The invention relates to a medical instrument, and specifically to a positioning device and a positioning method for a mechanical arm of a surgical robot.

### Background Art

Laparoscopic surgical robots have been widely used in minimally invasive abdominal surgery and usually use three or four mechanical arms to replace human arms to complete complex surgery. During surgery, usually, several small holes are firstly formed in a patient's abdominal wall, then poking cards are inserted into the human body through the small holes, afterwards mechanical arms are placed in suitable positions, and the poking cards are sleeved on the different mechanical arms, so as to realize the follow-up of the poking cards and the mechanical arms. However, during passive positioning, it is difficult to accurately align the mechanical arms with positions of the poking cards to make the poking cards coaxial with the entry direction of the mechanical arms, so that every time the poking cards and the mechanical arms will be disassembled after their locking to be adjusted. The adjustment process brings the risk of secondary trauma to the patient's abdominal wall wound, and in severe cases the wound may be torn, threatening a patient's life.

### Summary of the Invention

In order to solve one of the above problems, the present invention provides a positioning device and a positioning method for a mechanical arm of a surgical robot.

A positioning device for a mechanical arm of a surgical robot in the invention includes a poking card holder for being connected to an end of a mechanical arm, and an auxiliary positioning unit detachably connected to the poking card holder; and the auxiliary positioning unit is used to simulate a part of a surgical poking card located above a surgical poking card identification line to determine an accurate position where the mechanical arm is connected with the surgical poking card.

Alternatively, the auxiliary positioning unit includes a positioning end; and, the distance from the connection between the auxiliary positioning unit and the poking card holder to an end of the positioning end away from the connection, and the distance from the connection between the surgical poking card and the poking card holder to the surgical poking card identification line, satisfy a preset relationship.

Alternatively, the auxiliary positioning unit further includes a connecting end, the connecting end and the positioning end are connected with each other, and the connecting end is used for being held in the poking card holder, and one end of the positioning end away from the connecting end is in a shape of smooth spherical surface.

Alternatively, the poking card holder includes a base, a first collet and a second collet arranged opposite to each other, a pull rod device and a pull rod control device.

The first collet and the second collet are both connected to the base, and are suitable for rotating relative to the base.

The pull rod device is connected with the first collet and the second collet, and is used to drive the first collet and the second collet to be clamped or loosened relative to each other.

The pull rod control device cooperates with the pull rod device to lock or release the pull rod device when the first collet and the second collet are clamped relative to each other.

The connecting end of the auxiliary positioning unit is used to be held between the first collet and the second collet.

Alternatively, the pull rod device includes a connecting plate and a pull rod whose one end is a hinged end and whose other end is an installation end, and the installation end of the pull rod is installed on the base.

The first collet and the second collet each have one end which is a free end, and the first collet and the second collet each have the other end which is connecting end, and connections where the first collet and the second collet are connected with the base are respectively located between their respective free ends and connecting ends.

One end of the connecting plate is hinged with the connecting ends of the first collet and the second collet respectively, and the other end of the connecting plate is hinged with the hinged end of the pull rod.

The pull rod control device is used to lock or release the pull rod.

Alternatively, a baffle is provided on one side of the pull rod and protrudes from the side.

The pull rod control device is a rod-shaped member and is provided with a pull rod accommodating groove and a baffle accommodating groove, with the pull rod accommodating groove having a greater width than the pull rod. The pull rod control device is provided between two opposite sides of the base, is perpendicular to the pull rod, and is suitable to move between the two opposite sides relative to the base.

The pull rod is held between the pull rod control device and the base and passes through the pull rod accommodating groove. The baffle is suitable for being accommodated in the baffle accommodating groove to be blocked by a side wall of the baffle accommodating groove.

Alternatively, a first automatic reset device is provided between the installation end of the pull rod and the base, a second automatic reset device is provided between the pull rod control device and the base, a limit block is provided on the base, and the limit block is provided with an installation space for the pull rod to pass through.

The pull rod passes through the installation space, and the pull rod is suitable for sliding relative to the limit block between the direction close to the first collet and the second collet and the direction away from the first collet and the second collet.

Alternatively, a left side wall is formed on a left side of the base, a right side wall is formed on a right side of the base, and one end of the pull rod control device is a control end. The left side wall is provided with an installation hole which the control end passes through to extend beyond the base. The other end of the pull rod control device is an installation end, the installation end is provided on the right side wall which is provided with a guide groove, the guide groove is suitable for accommodating the installation end of the pull rod control device, and the second automatic reset device is provided in the guide groove.

A positioning method for a mechanical arm of a surgical robot, which uses the above positioning device for a mechanical arm of a surgical robot, comprises the steps of:
swinging the mechanical arm for a bottom center of the auxiliary positioning unit connected to the poking car holder of the mechanical arm to coincide with a surface center of the surgical poking card identification line; and
removing the auxiliary positioning unit, and connecting the surgical poking card located below the surgical poking card identification line with the poking card holder.

Alternatively, the step of swinging the mechanical arm for a bottom center of the auxiliary positioning unit connected to the poking car holder of the mechanical arm to coincide with a surface center of the surgical poking card identification line, comprises: installing the auxiliary positioning unit on the poking card holder of the mechanical arm; and, making the bottom center of the auxiliary positioning unit coincide with the surface center of the surgical poking card identification line.

Alternatively, one end of the auxiliary positioning unit facing the surgical poking card identification line is in a shape of smooth spherical surface. The step of making the bottom center of the auxiliary positioning unit coincide with the surface center of the surgical poking card identification line, comprises: making an apex of the smooth spherical surface coincide with the surface center of the surgical poking card identification line.

Compared with the prior art, the invention has beneficial effects that, due to the detachable connection of the auxiliary positioning unit of the positioning device for a mechanical arm of a surgical robot in the invention to the poking card holder, after the adjustment of the mechanical arm to enable the auxiliary positioning unit to simulate the position of the surgical poking card, the mechanical arm is positioned and then the mechanical arm and the surgical poking card are locked, avoiding the disassembly of the mechanical arm and the surgical poking card after their locking to adjust the position of the mechanical arm, avoiding the risk of secondary trauma to a patient's abdominal wall wound during the adjustment process, and realizing the quick connection between the mechanical arm and the surgical poking card, which greatly shortens the preparation time before surgery.

### Brief Description of the Drawings

FIG. 1 is a structure diagram of a positioning device for a mechanical arm of a surgical robot in the invention.
FIG. 2 is a schematic diagram of a use state of surgical poking cards used for being connected with mechanical arms of a surgical robot.
FIG. 3 is a structural diagram of an auxiliary positioning unit in a positioning device for a mechanical arm of a surgical robot in the invention.
FIG. 4 is an axial view of a poking card holder in a positioning device for a mechanical arm of a surgical robot in the invention.
FIG. 5 is a front view of a clamped state of a poking card holder in a positioning device for a mechanical arm of a surgical robot in the invention.
FIG. 6 is a front view of a loosened state of a poking card holder in a positioning device for a mechanical arm of a surgical robot in the invention.
FIG. 7 is a structure diagram of a pull rod of a poking card holder in a positioning device for a mechanical arm of a surgical robot in the invention.
FIG. 8 is a structure diagram of a pull rod control device of a poking card holder in a positioning device for a mechanical arm of a surgical robot in the invention.
FIG. 9 is a flow chart of a positioning method for a mechanical arm of a surgical robot in the invention.
FIG. 10 is a schematic diagram of the process of swinging a mechanical arm in the positioning method for a mechanical arm of a surgical robot in the invention.

### Description of Reference Numerals

poking card holder; 02-mechanical arm; 03-auxiliary positioning unit; 04-surgical poking card; 11-base; 111-installation groove; 112-lefe side wall; 113-right side wall; 114-bottom wall; 115-guide groove; 12-first collet; 13-connecting plate; 14-pull rod; 141-first automatic reset device; 142-baffle; 15-pull rod control device; 151-pull rod accommodating groove; 152-baffle accommodating groove; 153-second automatic reset device; 16-limit block.

### Detailed Description of the Invention

The above and other technical features and advantages of the invention will be described in more detail below in combination with the accompanying drawings.

In addition, in the description of the invention, it should be understood that the forward direction of "X" in the accompanying drawings represents the left side, and correspondingly the reverse direction of "X" represents the right side; the forward direction of "Y" represents the front, and accordingly the reverse direction of "Y" represents the rear; and, the forward direction of "Z" represents the above, and correspondingly the reverse direction of "Z" represents the below. The orientation or positional relationship indicated by the terms "X", "Y", "Z", etc., is only for facilitating the description of the invention and the simplification of the description, rather than indicating or implying that the referred devices or elements must have a specific orientation, and be constructed and operated in a specific orientation. Therefore, it cannot be understood as a limitation of the invention.

If there are descriptions involving "first", "second", etc., in embodiments of the invention, the descriptions of "first", "second", etc., are only for the purpose of description, and should not be construed as indicating or implying their relative importance or implicitly indicating the number of the indicated technical features.

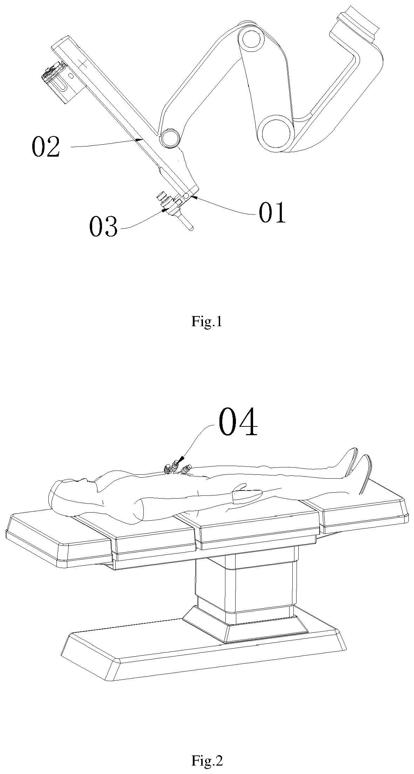

As shown in FIG. 1, a positioning device for a mechanical arm of a surgical robot according to an embodiment of the invention, includes a poking card holder 01 for being connected to an end of a mechanical arm 02, and an auxiliary positioning unit 03 detachably connected to the poking card holder 01. As shown in FIG. 2, the auxiliary positioning unit 03 is used to simulate a part of a surgical poking card 04 located above a patient's surgical poking card identification line to determine a position of the mechanical arm 02, so that the mechanical arm 02 is connected with the surgical poking card 04 at a suitable position. Due to the detachable connection of the auxiliary positioning unit 03 to the poking card holder 01, after the adjustment of the mechanical arm 02 to enable the auxiliary positioning unit 03 to simulate a position of the surgical poking card 04, the mechanical arm 02 is positioned and then the mechanical arm 02 and the surgical poking card 04 are locked, avoiding the disassembly of the mechanical arm 02 and the surgical poking card 04 after their locking to adjust the position of the mechanical arm 02, avoiding the risk of secondary trauma to a patient's abdominal wall wound during the adjustment process, and realizing the quick connection between the mechanical arm 02 and the surgical poking card 04, which greatly shortens the preparation time before surgery.

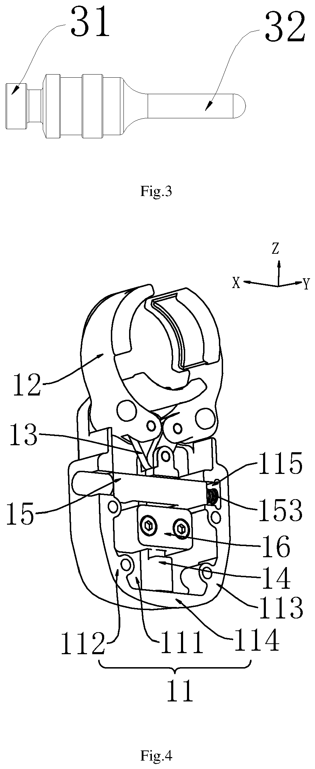

In some embodiments, as shown in FIG. 3, the auxiliary positioning unit 03 includes a connecting end 31 and a positioning end 32 which are integrally formed, with the connecting end 31 for being held on the poking card holder 01, and with an end of the positioning end 32 away from the connection end 31 in a shape of smooth spherical surface. The distance from the connection between the connection end 31 of the auxiliary positioning unit 03 and the poking card holder 01 to the end of the positioning end 32 away from the connection end 31, and the distance from the connection between the surgical poking card 04 and the poking card holder 01 to a surface portion of the surgical poking card identification line, satisfy a preset relationship. In some specific embodiments, when the auxiliary positioning unit 03 is arranged in parallel with the surgical poking card 04, assuming that the distance from the connection between the connection end 31 of the auxiliary positioning unit 03 and the poking card holder 01 to the end of the positioning end 32 away from the connection end 31 is L1, and the distance from the connection between the surgical poking card 04 and the poking card holder 01 to the surface portion of the surgical poking card identification line is L2, then L1 is equal to L2. Specifically, the auxiliary positioning unit 03 can use a positioning poking card which has a smaller length than the surgical poking card 02. Since the end of the positioning end 32 away from the connecting end 31 is in a shape of smooth spherical surface, and L1 is equal to L2, when an apex of the smooth spherical surface at the end of the positioning end 32 is made to coincide with a surface center of the surgical poking card identification line of a patient's abdominal wall, the auxiliary positioning unit 03 can simulate the position of the surgical poking card 04 more accurately, and the position of the mechanical arm 02 determined thereby is more accurate.

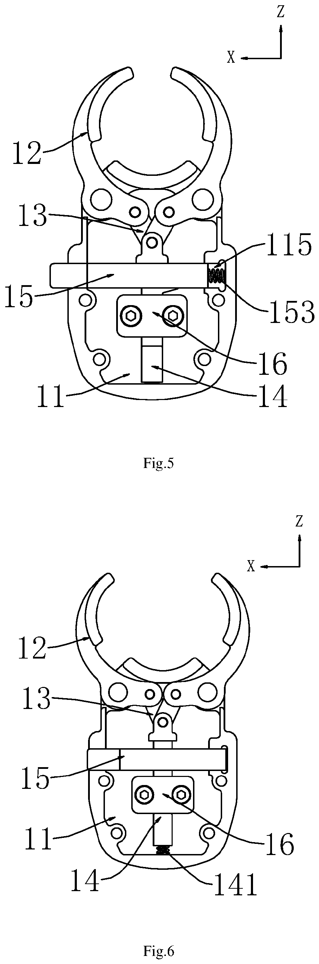

In some embodiments, as shown in FIGS. 4, 5, and 6, the poking card holder 01 includes a base 11, a first collet 12, a second collet, a pull rod device and a pull rod control device. The first collet 12 and the second collet are both connected to the base 11, with the first collet 12 arranged opposite to the second collet, and the first collet 12 and the second collet are suitable for rotation relative to the base 11. The pull rod device is connected with both the first collet 12 and the second collet, and can drive the first collet 12 and the second collet to be clamped or loosened relative to each other. The pull rod control device cooperates with the pull rod device to lock or release the pull rod device when the first collet 12 and the second collet are clamped relative to each other. The connecting end 31 of the auxiliary positioning unit 03 is used to be held between the first collet 12 and the second collet.

The pull rod device can drive the first collet 12 and the second collet to be clamped or loosened, and in the clamped state, the pull rod control device can lock the pull rod device to ensure that the clamped state of the first collet 12 and the second collet remains stable, thus avoiding accidental injury to a patient and facilitating operation.

Specifically, the pull rod device includes a connecting plate 13 and a pull rod 14 whose one end is a hinged end and whose other end is an installation end, and the installation end of the pull rod 14 is installed on the base 11.

The first collet 12 and the second collet each have one end which is a free end, and each have the other end which is a connecting end, and the connections where the first collet 12 and the second collet are connected to the base 11 are respectively located between their respective free ends and connecting ends.

One end of the connecting plate 13 is hinged with the connecting ends of the first collet 12 and the second collet respectively, and the other end of the connecting plate 13 is hinged with the hinged end of the pull rod 14. The pull rod 14 is pulled to move away from the first collet 12 and the second collet, and then the pull rod 14 drives the first collet 12 and the second collet to rotate relative to the base 11, by the connecting plate 13, so that the free ends of the first collet 12 and the second collet are close to each other, with the first collet 12 and the second collet clamped. The pull rod 14 is pulled to move close to the first collet 12 and the second collet, and then the pull rod 14 drives the first collet 12 and the second collet to rotate relative to the base 11, by the connecting plate 13, so that the free ends of the first collet 12 and the second collet are away from each other, with the first collet 12 and the second collet loosened.

The pull rod control device 15 cooperates with the pull rod 14 and is used to lock or release the pull rod 14.

The pull rod device can drive the first collet and the second collet to be clamped or loosened, and in the clamped state, the pull rod control device can lock the pull rod to ensure that the clamped state of the first collet 12 and the second collet remains stable, thus avoiding accidental injury to a patient and facilitating operation.

In some embodiments, a first automatic reset device 141 is provided between the installation end of the pull rod 14 and the base 11, and specifically, the first automatic reset device 141 uses a spring. After the pull rod control device 15 releases the pull rod 14, the pull rod 14 can be automatically reset, which makes operation more convenient.

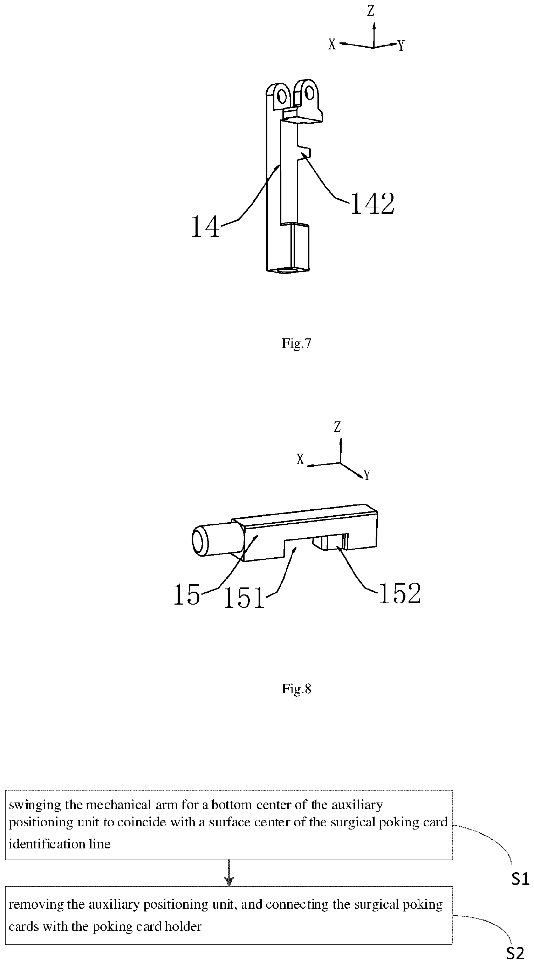

Specifically, as shown in FIG. 4, the base 11 is a plate-shaped member, and a left side and a right side of the base 11 correspond to each other. As shown in FIGS. 7 and 8, a baffle 142 is provided on one side of the pull rod 14 and protrudes from the side. The pull rod control device 15 is a rod-shaped member, whose one end is an installation end and whose other end 15 is a control end, and the installation end and the control end of the pull rod control device 15 are respectively provided on the two corresponding sides of the base 11. Moreover, the pull rod control device 15 and the pull rod 14 are perpendicular to each other, the pull rod control device 15 can move between the two corresponding sides relative to the base 11. The pull rod control device 15 is provided with a pull rod accommodating groove 151 and a baffle accommodating groove 152, with the pull rod accommodating groove 151 having a greater width than the pull rod 14, where the width refers to a dimension along the X-axis direction of the coordinate axis. The pull rod accommodating groove 151 and the baffle accommodating groove 152 communicate with each other. The pull rod 14 is held between the pull rod control device 15 and the base 11, and passes through the pull rod accommodating groove 151. The pull rod 14 is suitable to move close to or away from the first collet 12 and the second collet relative to the pull rod control device 15. When the pull rod control device 15 is located on the left side, the baffle 142 is accommodated in the baffle accommodating groove 152 and is blocked by a side wall of the baffle accommodating groove 152, thereby locking the pull rod 14. When the pull rod control device 15 is located on the right side, the baffle 142 is disengaged from the baffle accommodating groove 152, thereby releasing the pull rod 14.

In some embodiments, a second automatic reset device 153 is provided between the installation end of the pull rod control device 15 and the base 11, and specifically, the second automatic reset device 153 uses a spring. Due to the arrangement of the second automatic reset device 153, when the force acting on the control end of the pull rod control device 15 disappears, the pull rod 14 can be automatically reset, which makes operation more convenient.

In some embodiments, the base 11 is provided with a limit block 16 which is located on a side of the pull rod control device 15 close to the installation end of the pull rod 14, and the limit block 16 is provided with an installation space for the pull rod 14 to pass through. Specifically, the limit block 16 and the base 11 are connected by bolts, and the installation space can be a through hole provided in the limit block 16 or can also be a groove provided in a side of the limit block 16 facing the base 11. The pull rod 14 passes through the installation space in the limit block 16, and the pull rod 14 is suitable to slide relative to the limit block 16 between the direction close to the first collet 12 and the second collet and the direction away from the first collet 12 and the second collet. Due to the arrangement of the limit block 16, the pull rod 14 is restricted to move only close to or away from the first collet 12 and the second collet so as to prevent the pull rod 14 from moving left and right, which makes the poking card holder 01 more stable in the clamped state, avoiding the injury of a patient during surgery.

Specifically, the first collet 12 and the second collet are both in a semicircular arc shape. An installation groove 111 is formed in the middle of a side surface of the base 11, a left side wall 112 is formed on the left side of the base 11, a right side wall 113 is formed on the right side of the base 11, and a bottom wall 114 is formed at the bottom of the base 11. The pull rod 14, the limit block 16 and the pull rod control device 15 are all provided in the installation groove 111, the control end and the installation end of the pull rod control device 15 are respectively provided on the left side wall 112 and the right side wall 113 of the base 11, and the installation end of the pull rod 14 is installed on the bottom wall 114. As shown in FIG. 1, the left side wall 112 is provided with an installation hole which the control end of the pull rod control device 15 passes through to extend beyond the base 11. The right side wall 113 is provided with a guide groove 115 in which a spring is provided, and the guide groove 115 is suitable for accommodating the installation end of the pull rod control device 15 to move left and right the pull rod control device 15 relative to the base 11. The extension of the control end beyond the base 11 makes operation more convenient, and the arrangement of the guide groove 115 limits the movement direction of the pull rod control device 15 in a direction perpendicular to the pull rod 14, making the poking card holder 01 more stable in the clamped state.

In use, the poking card holder 01 opens the first collet 12 and the second collet to sleeve them on the auxiliary positioning unit 03, the first collet 12 and the second collet are pressed with your fingers, the pull rod 14 is pulled so as to move the pull rod 14 down and make the first automatic reset device 141 in a compressed state, and then the second automatic reset device 153 is ejected out to accommodate the baffle 142 of the pull rod 14 in the baffle accommodating groove 152 of the pull rod control device 15, thereby clamping the first collet 12 and the second collet on the auxiliary positioning unit 03. At the completion of positioning, the control end of the pull rod control device 15 is pressed to compress the second automatic reset device 153, the pull rod control device 15 is pressed to move it right so as to disengage the baffle 142 of the pull rod 14 from the baffle accommodating groove 152 of the pull rod control device 15, and the first automatic reset device 141 is ejected out to push the pull rod 14 to move up, thereby loosening the first collet 12 and the second collet from the auxiliary positioning unit 03.

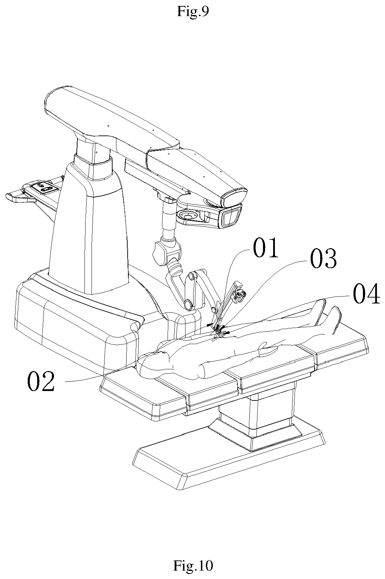

There are 3-4 surgical holes in a patient's abdominal wall at positions corresponding to the surgery, with the surgical poking cards 04 inserted into the surgical holes. A positioning method for a mechanical arm of a surgical robot according to an embodiment of the invention, as shown in FIG. 9, comprises the following steps of:
S1: swinging the mechanical arm 02 for a bottom center of the auxiliary positioning unit 03 connected to the poking car holder 01 at the end of the mechanical arm 02 to coincide with the surface center of the surgical poking card identification line.

Specifically, the connecting end 31 of the auxiliary positioning unit 03 is held on the poking card holder 01 on the mechanical arm 02 required to be positioned, the mechanical arm 02 is swung for the bottom center of the auxiliary positioning unit 03 connected to the poking car holder 01 at the end of the mechanical arm 02 to coincide with the surface center of the surgical poking card identification line, and the mechanical arm 02 is positioned to keep its position unchanged. As shown in FIG. 10, the length of the auxiliary positioning unit 03 is equal to the length of the part of the surgical poking card 04 located above the surgical poking card identification line, and at this time, the position of the mechanical arm 02 is an accurate position where the mechanical arm 02 is suitable to be connected with the surgical poking card 04.

Specifically, an end of the positioning end 32 of the auxiliary positioning unit 03 facing the surgical poking card identification line is in a shape of smooth spherical surface. In the process of swinging the mechanical arm 02 for the bottom center of the auxiliary positioning unit 03 connected to the poking car holder 01 to coincide with the surface center of the surgical poking card identification line, the apex of the smooth spherical surface of the positioning end 32 is made to coincide with the surface center of the surgical poking card identification line.

S2: removing the auxiliary positioning unit 03, and connecting the surgical poking card 04 located below the surgical poking card identification line with the poking card holder 01.

After the above steps S1 to S2 are used to complete the positioning of each mechanical arm 02 in sequence, the surgery can be started.

It is necessary to explain that the positioning method for a mechanical arm of a surgical robot in the embodiment is actually based on the use method for the positioning device for a mechanical arm of a surgical robot, and the positioning device for a mechanical arm of a surgical robot is used for positioning before performing surgery. For example, the positioning method for a mechanical arm of a surgical robot in the embodiment can use an experimental dummy or training equipment as an implementation object. The positioning device for a mechanical arm of a surgical robot in the embodiment is used for positioning to determine a target position before surgery, and the surgery is started when a patient's affected area is placed in the target positon during surgery.

By swinging the mechanical arm 02 for the bottom center of the auxiliary positioning unit 03 connected to the poking car holder 01 at the end of the mechanical arm 02 to coincide with the surface center of the surgical poking card identification line, locking the mechanical arm 02 and the surgical poking card 04 after the determination of the accurate position where the mechanical arm 02 is suitable to be connected with the surgical poking card 04 and the positioning of the mechanical arm 02, a positioning method for a mechanical arm of a surgical robot in the invention can avoid the disassembly of the mechanical arm 02 and the surgical poking card 04 after their locking, avoid the risk of secondary trauma to a patient's abdominal wall wound during the adjustment process, and realize the quick connection between the mechanical arm 02 and the surgical poking card 04, which greatly shortens the preparation time before surgery.

Although the present disclosure is disclosed above, the protection scope of the present disclosure is not limited thereto. Those skilled in the art can make various changes and modifications without departing from the spirit and scope of the present disclosure, and all these changes and modifications will fall within the protection scope of the present invention.

## Claims

1. A positioning device for a mechanical arm of a surgical robot, **characterized in that** the positioning device includes a poking card holder (01) for being connected to an end of a mechanical arm (01), and an auxiliary positioning unit (03) detachably connected to the poking card holder (01); and the auxiliary positioning unit (03) is used to simulate a part of a surgical poking card (04) located above a surgical poking card identification line to determine an accurate position where the mechanical arm (02) is connected with the surgical poking card (04).

2. The positioning device for a mechanical arm of a surgical robot according to Claim 1, **characterized in that** the auxiliary positioning unit (03) includes a positioning end (32); and, the distance from the connection between the auxiliary positioning unit (03) and the poking card holder (01) to an end of the positioning end (32) away from the connection, and the distance from the connection between the surgical poking card (04) and the poking card holder (01) to the surgical poking card identification line, satisfy a preset relationship.

3. The positioning device for a mechanical arm of a surgical robot according to Claim 1, **characterized in that** the auxiliary positioning unit (03) further includes a connecting end (31), the connecting end (31) and the positioning end (32) are connected with each other, the connecting end (31) is used for being held in the poking card holder (01), and one end of the positioning end (32) away from the connecting end (31) is in a shape of smooth spherical surface.

4. The positioning device for a mechanical arm of a surgical robot according to Claim 1, **characterized in that** the poking card holder (01) includes a base (11), a first collet (12) and a second collet arranged opposite to each other, a pull rod device and a pull rod control device (15);
the first collet (12) and the second collet are both connected to the base (11), and are suitable for rotating relative to the base (11);
the pull rod device is connected with the first collet (12) and the second collet, and is used to drive the first collet (12) and the second collet to be clamped or loosened relative to each other;
the pull rod control device (15) cooperates with the pull rod device to lock or release the pull rod device when the first collet (12) and the second collet are clamped relative to each other; and
the connecting end (31) of the auxiliary positioning unit (03) is used for being held between the first collet (12) and the second collet.

5. The positioning device for a mechanical arm of a surgical robot according to Claim 4, **characterized in that** the pull rod device includes a connecting plate (13) and a pull rod (14) whose one end is a hinged end and whose other end is an installation end, and the installation end of the pull rod (14) is installed on the base (11);
the first collet (12) and the second collet each have one end which is a free end, and the first collet (12) and
the second collet each have the other end which is a connecting end; and connections where the first collet (12) and the second collet are connected with the base (11) are respectively located between their respective free ends and connecting ends;
one end of the connecting plate (13) is hinged with the connecting ends of the first collet (12) and the second collet respectively, and the other end of the connecting plate (13) is hinged with the hinged end of the pull rod (14); and
the pull rod control device (15) is used to lock or release the pull rod (14).

6. The positioning device for a mechanical arm of a surgical robot according to Claim 5, **characterized in that** a baffle (142) is provided on one side of the pull rod (14) and protrudes from the side;
the pull rod control device (15) is a rod-shaped member and is provided with a pull rod accommodating groove (151) and a baffle accommodating groove (152), with the pull rod accommodating groove (151) having a greater width than the pull rod (14); the pull rod control device (15) is provided between two opposite sides of the base (11), is perpendicular to the pull rod (14), and is suitable to move between the two opposite sides relative to the base (11); and
the pull rod (14) is held between the pull rod control device (15) and the base (11) and passes through the pull rod accommodating groove (151); the baffle (142) is suitable for being accommodated in the baffle accommodating groove (152) to be blocked by a side wall of the baffle accommodating groove (152).

7. The positioning device for a mechanical arm of a surgical robot according to Claim 6, **characterized in that** a first automatic reset device (141) is provided between the installation end of the pull rod (14) and the base (11), a second automatic reset device (153) is provided between the pull rod control device (15) and the base (11), a limit block (16) is provided on the base (11), and the limit block (16) is provided with an installation space for the pull rod (14) to pass through; and
the pull rod (14) passes through the installation space, and the pull rod (14) is suitable for sliding relative to the limit block (16) between the direction close to the first collet (12) and the second collet and the direction away from the first collet (12) and the second collet.

8. The positioning device for a mechanical arm of a surgical robot according to Claim 7, **characterized in that** one end of the pull rod control device (15) is a control end, a left side wall (112) is formed on a left side of the base (11), and a right side wall (113) is formed on a right side of the base (11); the left side wall (112) is provided with an installation hole which the control end passes through to extend beyond the base (11); and, the other end of the pull rod control device (15) is an installation end, the installation end is provided on the right side wall (113) which is provided with a guide groove (115), the guide groove (115) is suitable for accommodating the installation end of the pull rod control device (15), and the second automatic reset device (153) is provided in the guide groove (115).

9. A positioning method for a mechanical arm of a surgical robot, which uses the positioning device for a mechanical arm of a surgical robot according to any one of Claims 1-8, **characterized in that** the positioning method comprises the steps of:
swinging the mechanical arm (02) for a bottom center of the auxiliary positioning unit (03) connected to the poking car holder (01) of the mechanical arm (02) to coincide with a surface center of the surgical poking card identification line; and
removing the auxiliary positioning unit (03), and connecting the surgical poking card (04) located below the surgical poking card identification line with the poking card holder (01).

10. The positioning method for a mechanical arm of a surgical robot according to Claim 9, **characterized in that** the step of swinging the mechanical arm (02) for a bottom center of the auxiliary positioning unit (03) connected to the poking car holder (01) of the mechanical arm (02) to coincide with a surface center of the surgical poking card identification line, comprises:
installing the auxiliary positioning unit (03) on the poking card holder (01) of the mechanical arm (02); and making the bottom center of the auxiliary positioning unit (03) coincide with the surface center of the surgical poking card identification line.

11. The positioning method for a mechanical arm of a surgical robot according to Claim 10, **characterized in that** an end of the auxiliary positioning unit (03) facing the surgical poking card identification line is in a shape of smooth spherical surface; and the step of making the bottom center of the auxiliary positioning unit (03) coincide with the surface center of the surgical poking card identification line, comprises:
making an apex of the smooth spherical surface coincide with the surface center of the surgical poking card identification line.
